# EUROPÄISCHE PATENTSCHRIFT

(11) **EP 1 713 765 B1**
(45) Veröffentlichungstag und Bekanntmachung des Hinweises auf die Patenterteilung: **14.09.2011**
(21) Anmeldenummer: 05701206.4
(22) Anmeldetag: 27.01.2005
(51) Int. Cl.: C07C 253/30, C07C 255/07

(54) **ISOMERISIERUNG VON CIS-2-PENTENNITRIL ZU 3-PENTENNITRIL IN EINER REAKTIVDESTILLATION**
ISOMERISATION OF CIS-2-PENTENENITRILE TO FORM 3-PENTENENITRILE IN A REACTIVE DISTILLATION
ISOMERISATION DE CIS-2-PENTENE NITRILE EN 3-PENTENE NITRILE DANS UNE DISTILLATION REACTIVE

(30) Priorität: 29.01.2004 DE 102004004716
(43) Veröffentlichungstag der Anmeldung: 25.10.2006
(73) Patentinhaber: BASF SE, 67056 Ludwigshafen (DE)
(72) Erfinder: SCHEIDEL, Jens, 69493 Hirschberg (DE); JUNGKAMP, Tim, B-2950 Kapellen (BE); BARTSCH, Michael, 67433 Neustadt (DE); HADERLEIN, Gerd, 67269 Grünstadt (DE); BAUMANN, Robert, 68159 Mannheim (DE); LUYKEN, Hermann, 67069 Ludwigshafen (DE)
(86) Internationale Anmeldenummer: PCT/EP2005/000782
(87) Internationale Veröffentlichungsnummer: WO 2005/073177

(56) Entgegenhaltungen:
- WO-A-02/081417
- WO-A-2004/094364
- US-A- 3 526 654

## Beschreibung

Die vorliegende Erfindung betrifft ein Verfahren zur Isomerisierung von Pentennitril in einem Eduktstrom.

Adipodinitril ist ein wichtiges Ausgangsprodukt in der Nylonherstellung, das durch zweifache Hydrocyanierung von 1,3-Butadien erhalten wird. In der ersten Hydrocyanierung wird das 1,3-Butadien zu 3-Pentennitril hydrocyaniert, wobei als Nebenprodukte hauptsächlich cis-2-Pentennitril, 2-Methyl-2-butennitril, 2-Methyl-3-butennitril, C₉-Nitrile und Methylglutarnitril erhalten werden. In einer zweiten, sich anschließenden Hydrocyanierung wird 3-Pentennitril mit Cyanwasserstoff zu Adipodinitril umgesetzt. Beide Hydrocyanierungen werden durch Nickel(0)-Phosphor-Komplexe katalysiert. Dabei kann das cis-2-Pentennitril - im Gegensatz zu 3-Pentennitril, beispielsweise trans-3-Pentennitril - in Gegenwart der Nickel(0) enthaltenden Katalysatoren nicht zu Adipodinitril hydrocyaniert werden. Hierdurch wird die Ausbeute der Adipodinitril-Synthese herabgesenkt.

Wünschenswert ist es demnach, cis-2-Pentennitril zu trans-3-Pentennitril zu isomerisieren, um dieses dann wieder in die Adipodinitril-Synthese zurückführen zu können.

US 3,526,654 offenbart die Isomerisierung von cis-2-Pentennitril zu trans-3-Pentennitril in Gegenwart von Siliziumdioxid, Aluminiumoxid oder Natrium-Calcium-Silikat, wobei die Katalysatoren in verschiedenen Modifikationen vorliegen. Die Isomerisierung wird in der Flüssig- oder Gasphase bei Temperaturen von 25 °C bis 500 °C durchgeführt. Dieses Verfahren ist aufgrund eines geringen Umsatzes und einer langen Isomerisierungsdauer unwirtschaftlich. Im Allgemeinen kann die Geschwindigkeit einer Isomerisierung durch eine Erhöhung der Reaktionstemperatur angehoben werden. Dieses ist in der vorliegenden Isomerisierung von cis-2-Pentennitril zu trans-3-Pentennitril aber nicht zweckdienlich, da im Fall von Pentennitrilen eine Erhöhung der Reaktionstemperatur innerhalb des in der US 3,526,654 offenbarten Temperaturbereichs zu einer Bildung einer technisch inakzeptablen hohen Menge an Oligomeren und Polymeren führt.

Die DE-A-103 23 803 beschreibt die Isomerisierung von cis-2-Pentennitril zu 3-Pentennitril an Aluminiumoxid als Katalysator. Hierbei werden im Allgemeinen Ausbeuten von 30 % bezüglich cis-2-Pentennitril erzielt. Wird der Umsatz an cis-2-Pentennitril erhöht, kommt es zu einer verstärkten Bildung von trans-2-Pentennitril gegenüber der gewünschten Bildung von trans-3-Pentennitril.

Der vorliegenden Erfindung liegt somit die Aufgabe zugrunde, ein Verfahren bereitzustellen, das die Isomerisierung insbesondere von cis-2-Pentennitril zu trans-3-Pentennitril mit Umsätzen bezüglich des Isomerisierungsedukts ermöglicht, die wirtschaftlich vertretbar sind. Dabei soll gleichzeitig eine hohe Raum-Zeit-Ausbeute von trans-3-Pentennitril bezüglich cis-2-Pentennitril erreicht werden.

Die Aufgabe der vorliegenden Erfindung wird gelöst durch ein Verfahren zur Isomerisierung von Pentennitrilen in einem Eduktstrom.

Das erfindungsgemäße Verfahren ist dadurch gekennzeichnet, dass die Isomerisierung an mindestens einem heterogenen Katalysator, ausgewählt aus der Gruppe der Oxide der 3. oder 4. Hauptgruppe oder der Oxide der 3. oder 4. Nebengruppe des Periodensystems der Elemente in einer Destillationskolonne, mindestens umfassend eine Sumpfzone, eine Reaktionszone und eine Kopfzone, stattfindet und dass während der Isomerisierung das Isomerisierungsedukt im Verhältnis zu dem Isomerisierungsprodukt in der Reaktionszone der Destillationskolonne destillativ angereichert wird.

In einer bevorzugten Ausführungsform der vorliegenden Erfindung wird cis-2-Pentennitril zu trans-3-Pentennitril isomerisiert.

Bei einer Isomerisierung von cis-2-Pentennitril kann der Eduktstrom weitere Bestandteile enthalten, die insbesondere ausgewählt sind aus der Gruppe, bestehend aus C5-Mononitrilen, C6-Dinitrilen, aliphatischen C1- bis C16-Alkanen, cyclischen C1- bis C16-Alkanen, aliphatischen C1- bis C16-Alkenen, cyclischen C1- bis C16-Alkenen, besonders bevorzugt ausgewählt aus der Gruppe, bestehend aus trans-3-Pentennitril, trans-2-Pentennitril, cis-3-Pentennitril, 4-Pentennitril, Z-2-Methyl-2-butennitril, E-2-Methyl-2-butennitril, 2-Methyl-3-butennitril, Methylglutarsäuredinitril, Ethylbernsteinsäuredinitril, Adipodinitril, Valeriansäurenitril, Cyclohexan, Methylcyclohexan, n-Heptan, n-Octan, Vinylcyclohexan, Ethylidencyclohexen und Vinylcyclohexen.

Der Eduktstrom stammt vorzugsweise aus einem Verfahren zur Hydrocyanierung von 3-Pentennitril.

Der Gehalt an cis-2-Pentennitril in dem Eduktstrom beträgt vorzugsweise 0,5 bis 100 Gew.-%, besonders bevorzugt 1,0 bis 98 Gew.-%, insbesondere 1,5 bis 97 Gew.-%.

Der in dem erfindungsgemäßen Verfahren eingesetzte Eduktstrom, der cis-2-Pentennitril enthält, fällt im Allgemeinen in an sich bekannten Verfahren an. Ein Beispiel hierfür ist ein Verfahren zur Hydrocyanierung von 3-Pentennitril, wobei unter 3-Pentennitril trans-3-Pentennitril, cis-3-Pentennitril, deren Gemische oder eine cis- bzw. trans-3-Pentennitril enthaltende Mischung verstanden wird. Alternativ kann der Eduktstrom aus einer Hydrocyanierung von 4-Pentennitril oder 4-Pentennitril enthaltenden Mischungen zu Adipodinitril stammen.

In einer bevorzugten Ausführungsform kann das erfindungsgemäße Verfahren in ein Hydrocyanierungsverfahren zur Herstellung von Adipodinitril integriert werden.

Das erfindungsgemäße Verfahren wird vorzugsweise in einer Destillationskolonne, mindestens umfassend eine Sumpfzone, eine Reaktionszone und eine Kopfzone, durchgeführt. Dabei sind Sumpfzone, Reaktionszone und Kopfzone in der vorgegebenen Reihenfolge von unten nach oben in der Destillationskolonne angeordnet. Es ist dabei nicht ausgeschlossen, dass auch in der Sumpf- oder Kopfzone noch Reaktion stattfindet

Zusätzlich kann die Destillationskolonne Einbauten mit destillativer Trennwirkung umfassen. Diese zusätzlichen Einbauten sind vorzugsweise unter- und/oder oberhalb der Reaktionszone angeordnet. In der unteren Trennzone, d.h. der Trennzone unterhalb der Reaktionszone, wird das schwer siedende Isomerisierungsprodukt weitgehend von leicht siedenden Komponenten abgetrennt. So werden z.B. trans-2-Pentennitril und trans-3-Pentennitril von nicht umgesetztem cis-2-Pentennitril getrennt. In der oberen Trennzone, d.h. der Trennzone oberhalb der Reaktionszone, werden leicht siedende Nebenkomponenten weitgehend von schwer siedenden Komponenten abgetrennt. So wird hier beispielsweise gegebenenfalls mit dem Eduktstrom eingetragenes E-2-Methyl-2-butennitril von trans-3-Pentennitril und trans-2-Pentennitril getrennt. Ebenso kann aus nicht isomerisierten cis-2-Pentennitril trans-3-Pentennitril und trans-2-Pentennitril abgereichert werden. Diese Trennungen sind nur beispielhaft aufgeführt und sind nicht einschränkend.

Bei optimaler Kolonnenkonfiguration kann so das gesamte cis-2-Pentennitril des Eduktstroms ohne zusätzlichen Reaktor umgesetzt werden und das gesamte trans-3-Pentennitril im Sumpf ohne zusätzlichen Trennapparat erhalten werden. Dabei sind die zusätzlichen Einbauten mit destillativer Trennwirkung (Trennzonen) im Allgemeinen von Vorteil, aber nicht zwingend notwendig. So kann auch eine der beiden oder beide Trennzonen entfallen.

Die Reaktionszone besteht im Allgemeinen aus mehreren unterschiedlichen Teilbereichen mit unterschiedlichen Funktionen. Die Teilbereiche unterscheiden sich durch die Aufgabe des Transportes von Gas zum Kopf der Kolonne und die Aufgabe des Ableitens von Flüssigkeit in Richtung des Kolonnensumpfes. Zudem können Flüssigkeitsverteiler innerhalb der Reaktionszone notwendig sein, um eine optimale Verteilung von Flüssigkeit über den Kolonnenquerschnitt zu gewährleisten. Auch Einbauten zum Eintragen von Wärme in die Kolonne können sich in der Reaktionszone befinden. Zudem sind vorzugsweise in den überwiegend Flüssigkeit führenden Teil als Katalysator geeignete Feststoffe eingebracht, wie sie im Folgenden beschrieben werden.

Die Isomerisierung von cis-2-Pentennitril zu trans-3-Pentennitril wird an einem heterogenen Katalysator durchgeführt.

Dabei erfolgt die Isomerisierung im Wesentlichen auf den Kolonneneinbauten der Reaktionszone oder an dem Feststoff, der als heterogener Katalysator im Sinne des erfindungsgemäßen Verfahrens geeignet ist und in Zwischenräume dieser Einbauten eingebracht wurde. Dabei werden die entstehenden Produkte, bevorzugt trans-3-Pentennitril, cis-3-Pentennitril und trans-2-Pentennitril, vorzugsweise gleichzeitig entfernt. Die Dimension der Reaktionszone richtet sich nach dem angestrebten Umsatzgrad und der Menge an cis-2-Pentennitril im Eduktstrom.

Der Katalysator kann in der Reaktionszone auf Böden mit hoher Verweilzeit der Flüssigkeit, beispielsweise Ventilböden, bevorzugt Glockenböden oder verwandte Bauarten, wie z.B. Tunnelböden oder Thormannböden, aufgebracht werden oder als Katalysatorschüttung in der Reaktionszone installiert werden. Es ist jedoch auch möglich, Katalysator enthaltende Packungen, beispielsweise Montz MULTIPAK oder Sulzer KATAPAK, zu verwenden oder den Katalysator in Form von Füllkörpern in die Kolonne einzubringen. Weiterhin ist es möglich, katalytisch aktive Destillationspackungen oder mit Katalysator gefüllte Gewebetaschen, sogenannte Bales oder Texas-Tea-Bags, zu verwenden.

Eine bevorzugte Ausführungsform ist die Verwendung von Katalysatorpackungen oder Füllkörpern, in die Katalysatorteilchen lose unter der Einwirkung der Schwerkraft eingebracht, verteilt und bei Bedarf wieder ausgetragen werden. Besonders bevorzugt ist dabei die Verwendung einer Katalysatorpackung oder von Füllkörpern, die erste und zweite Teilbereiche aufweisen, wobei in den ersten Teilbereichen der Packung die Katalysatorteilchen lose unter der Einwirkung der Schwerkraft eingebracht, verteilt und bei Bedarf wieder ausgetragen werden, und in den zweiten Teilbereichen jedoch aufgrund der geometrischen Verhältnisse im Vergleich zum Katalysatorteilchen keine Katalysatorteilchen eingebracht werden können. Dieses wird dadurch gewährleistet, dass in den ersten Teilbereichen der Quotient aus dem hydraulischen Durchmesser für den Gasstrom durch die Packung oder Füllkörper und dem äquivalenten Durchmesser der Katalysatorteilchen vorzugsweise im Bereich von 2 bis 20, besonders bevorzugt im Bereich von 5 bis 10, liegt, wobei die Katalysatorteilchen lose unter Einwirkung der Schwerkraft in die Zwischenräume eingebracht, verteilt und bei Bedarf ausgetragen werden und dass in den zweiten Teilbereichen der Quotient aus dem hydraulischen Durchmesser für den Gasstrom durch die Packung oder die Füllkörper und dem äquivalenten Durchmesser der Katalysatorteilchen kleiner als 1 ist und dass in die zweiten Teilbereiche keine Katalysatorteilchen eingebracht werden. Die Berechnung des hydraulischen Durchmessers für den Gasstrom durch die Packung ergibt sich dabei aus dem vierfachen der durchströmten Fläche geteilt durch den Umfang der Gaskanäle der strukturierten Packung. Die Berechnung des äquivalenten Partikeldurchmessers ergibt sich aus dem sechsfachen Volumen des Partikels geteilt durch die Oberfläche des Partikels (vgl. hierzu VDI Wärmeatlas, 5. Auflage, 1988 Lk1). Diese Katalysatorpackung ist in der Patentanmeldung WO 03/047747 A1 beschrieben, wobei die dort beschriebene Katalysatorpackung in die vorliegende Erfindung durch Bezugnahme eingeschlossen ist. Die zuvor beschriebene Katalysatorpackung ist besonders vorteilhaft, wenn das erfindungsgemäße Verfahren in der Flüssigphase durchgeführt wird.

In den Trennzonen der Destillationskolonne werden Einbauten mit destillativer Trennwirkung verwendet. Hierbei werden vorzugsweise Kolonneneinbauten mit hoher Trennstufenzahl wie Metallgewebepackungen oder Blechpackungen mit geordneter Struktur, beispielsweise Sulzer MELAPAK, Sulzer BX, Montz B1-Typen oder Montz A3-Typen, verwendet.

Zur Durchführung des erfindungsgemäßen Verfahrens verwendet man vorzugsweise Destillationskolonnen, die als, umfassend die Reaktions- und Trennzonen, 10 bis 100 Böden, besonders bevorzugt 10 bis 60 Böden, aufweisen. Als besonders vorteilhaft hat es sich erwiesen, wenn die Trennzone über der Reaktionszone 0 bis 50 Böden, vorzugsweise 2 bis 40 Böden, aufweist. Als besonders vorteilhaft hat es sich erwiesen, wenn die Reaktionszone 0 bis 100 Böden, vorzugsweise 2 bis 40 Böden, aufweist. Als besonders vorteilhaft hat es sich erwiesen, wenn die untere Trennzone 0 bis 50 Böden, vorzugsweise 5 bis 30 Böden, aufweist. Entsprechendes gilt für die sogenannten theoretischen Böden bei anderen Kolonneneinbauten.

Das erfindungsgemäße Verfahren der Isomerisierung von cis-2-Pentennitril wird vorzugsweise so durchgeführt, dass man das zu isomerisierende cis-2-Pentennitril oder ein solches enthaltendes Gemisch über einen oder mehrere Zuläufe, die je nach Gemischzusammensetzung unter-, inner- oder oberhalb der Reaktionszone liegen eindosiert.

Der Zulauf des Eduktstroms kann auch im Bereich der Einbauten, die nur destillative Trennwirkung, aber keine katalytische Aktivität aufweisen, erfolgen Dabei kann der Zulauf des Eduktstroms sowohl in die obere als auch in die untere Trennzone erfolgen. Ebenfalls ist es möglich, Edukströme über verschiedene Zuläufe innerhalb der gleichen oder unterschiedlicher Trennzonen und/oder der Reaktionszone einzudosieren.

In der Destillationskolonne werden Druck und Temperatur vorzugsweise so eingestellt, dass sich hohe Reaktionsgeschwindigkeiten bei ausreichend hoher Selektivität ergeben. Dabei wird der Druck in der Kopfzone vorzugsweise so eingestellt, dass die Temperatur in der Sumpfzone zwischen 30 und 300 °C, bevorzugt zwischen 40 und 250 °C, insbesondere zwischen 50 und 200 °C, beträgt. Die Verweilzeit in der Destillationskolonne beträgt vorzugsweise 1 Minute bis 10 Stunden, besonders bevorzugt 12 Minuten bis 3 Stunden.

Die optimalen Temperatur- und Druckverhältnisse werden im Allgemeinen durch die Einbindung der Isomerisierung in ein Verfahren, beispielsweise die zweifache Hydrocyanierung von 1,3-Butadien zu Adipodinitril, und die Arbeitstemperatur des Katalysators bestimmt. Die Einstellung des Drucks kann dabei mit einer Vakuumpumpe und/oder einer Druckregeleinrichtung geschehen, so dass die Druckverhältnisse den Anforderungen des Verfahrens angepasst sind.

Bei Verwendung von heterogenen Katalysatoren findet die Isomerisierung im Bereich der Reaktionszone der Destillationskolonne statt. Durch die überlagerte Destillation werden die gebildeten Reaktionsprodukte dem Reaktionsgleichgewicht und somit der Reaktionszone kontinuierlich entzogen, gelangen in den Sumpf der Destillationskolonne und werden vorzugsweise über einen Strom abgezogen. Die überlagerte Destillation sorgt somit für eine positive Beeinflussung des Reaktionsgleichgewichts in dem Sinne, dass stets das zu isomerisierende Pentennitril im Bereich der Reaktionszone angereichert und das isomerisierte Pentennitril und andere höher siedende Isomerisierungsprodukte bzw. -nebenprodukte, im Vergleich zu dem zu isomerisierenden Pentennitril, in der Reaktionszone abgereichert werden. Damit werden hohe Ausbeuten an isomerisierten Pentennitril bezüglich dem zu isomerisierenden Pentennitril erreicht.

Das höher siedende Isomerisierungsprodukt sammelt sich in der Sumpfzone der Destillationskolonne an und kann dort beispielsweise mittels einer Pumpe gegebenenfalls zusammen mit eventuell höher als das zu isomerisierende Pentennitril siedenden Nebenprodukten der Isomerisierung sowie gegebenenfalls bereits im Eduktstrom befindlichen höher als das zu isomerisierende Pentennitril siedenden Komponenten über einen Sumpfstrom abgezogen werden. Dabei kann in einer bevorzugten Ausführungsform der vorliegenden Erfindung ein Teil des Sumpfstroms über einen Verdampfer verdampft und über eine Brüdenleitung in die Destillationskolonne zurückgeführt werden. Hierdurch wird der für die Destillation erforderliche Brüden erzeugt.

Es ist auch möglich, zur Entfernung von leicht siedenden Komponenten zusätzlich ein Inertgas in die Sumpfzone der Destillationskolonne einzuspeisen. Beispiele für geeignete Inertgase sind Stickstoff oder Edelgase, wie Argon oder Helium oder Gemische derer oder dieses Gas enthaltende Gemische.

Am Kopf der Kolonne reichert sich nicht umgesetztes zu isomerisiererides Pentennitril und gegebenenfalls leichter als zu isomerisierendes Pentennitril siedende Komponenten aus dem Eduktstrom, gegebenenfalls zusammen mit leicht siedenden Nebenprodukten der Isomerisierung, an. In einer bevorzugten Ausführungsform der vorliegenden Erfindung wird dieser Kopfstrom über eine Leitung in einen Kondensator geführt, auskondensiert und über eine weitere Leitung ausgeschleust. Dabei kann vorzugsweise ein Teil des Kondensats wieder als Rücklauf in die Destillationskolonne gegeben werden. In einer bevorzugten Ausführungsform beträgt die Menge des Teiles des Kondensats, die wieder auf die Kolonne gegeben wird, mehr als 50 % des Kondensats, vorzugsweise mehr als 90 % des Kondensats. Auf diese Weise kann durch den internen Rücklauf in der Kolonne ein vorteilhaftes Konzentrationsprofil eingestellt werden.

Bevorzugt wird das Konzentrationsprofil in der Kolonne durch den Energieeintrag und das Rücklaufverhältnis so eingestellt, dass sich eine Anreicherung des zu isomerisierenden Pentennitrils am oberen Ende der Reaktionszone gegenüber dem unteren Ende der Reaktionszone ausbildet. So kann eine hohe Konzentration der leicht siedenden Reaktanden in der Reaktionszone eingestellt werden, die zu hohen Selektivitäten des bei niedrigen lokalen Umsätzen kinetisch bevorzugten Isomerisierungsproduktes trans-3-Pentennitril führt.

Vorzugsweise sollte ein Rücklaufverhältnis zwischen 1 und 3000, besonders bevorzugt zwischen 2 und 500, bezüglich der Zulaufmenge, eingestellt werden.

Zur Erhöhung der Verweilzeit in der Reaktionszone ist es möglich, einen Teilstrom durch eine oder mehrere Seitenabzüge aus der Destillationskolonne durch einen oder mehrere Behälter zu leiten und mit Hilfe jeweils einer Pumpe die diese Behälter verlassenden Teilströme wieder in die Kolonne zurückzuführen. Die Behälter können dabei mit heterogenem Katalysator gefüllt werden. Dabei ist eine bevorzugte Ausführungsform dadurch gegeben, dass die Behälter beheizt werden. Die Temperatur in den Behältern sollte vorzugsweise der Temperatur der Flüssigphase auf dem Abzugsboden entsprechen.

Es hat sich ferner als vorteilhaft erwiesen, wenn die Wärmezufuhr in das Destillationssystem, bestehend aus der Destillationskolonne und gegebenenfalls dem Behälter oder den Behältern, nicht nur über den Verdampfer, sondern zusätzlich über außenliegende Wärmetauscher oder über sich direkt auf den Kolonneneinbauten befindliche Wärmetauscher erfolgt.

Es ist zudem möglich, aus der Kolonne an beliebigen Stellen über Seitenabzüge Teilströme abzuziehen. So ist es beispielsweise auch möglich, die Kolonne unter totalem Rücklauf zu betreiben und über einen Seitenabzug unterhalb der Katalysatorpackung aber oberhalb des Zulaufs Zwischensieder im Siedebereich zwischen den zu isomerisierenden Pentennitril und dem isomerisierten Pentennitril auszuschleusen.

Das erfindungsgemäße Verfahren wird in Gegenwart mindestens eines Heterogenkatalysators durchgeführt. Darüber hinaus ist es auch möglich, die Isomerisierung in Gegenwart mindestens eines Heterogenkatalysators unter Zugabe mindestens eines Homogenkatalysators durchzuführen.

Als heterogener Katalysator wird ein Oxid der 3. oder 4. Hauptgruppe oder der 3. oder 4. Nebengruppe des Periodensystems der Elemente verwendet.

Erfindungsgemäß führt man die Isomerisierung vorzugsweise in Gegenwart von Aluminiumoxid als Heterogenkatalysator durch, wobei das Aluminiumoxid eine BET-Oberfläche von vorzugweise mindestens 50 m²/g, besonders bevorzugt mindestens 70 m²/g, insbesondere mindestens 100 m²/g, aufweist. Dabei sollte das Aluminiumoxid vorzugsweise eine BET-Oberfläche von höchstens 400 m²/g, besonders bevorzugt höchstens 350 m²/g, insbesondere höchstens 300 m²/g, aufweisen.

Unter der BET-Oberfläche wird im Sinne der vorliegenden Erfindung die spezifische Oberfläche verstanden, die durch Messung der physiosorbierten Gasmenge nach dem in Brunauer, Emmett, Teller, J. Am. Chem. Soc. 60 (1938), Seite 309 beschriebenen Verfahrens bestimmt wird.

Das Aluminiumoxid kann gegebenenfalls in reiner Form vorliegen.

Es ist aber auch möglich, Aluminiumoxid einzusetzen, das weitere Verbindungen umfasst, beispielsweise Seltenerdenoxide, wie Ceroxid, Praeseodymoxid, Siliziumdioxid, Titandioxid, Eisenoxid, Alkalioxide, Erdalkalioxide oder deren Gemische. Dabei können solche Verbindungen vorzugsweise in Mengen von 10 Gew.-ppm bis 10 Gew.%, besonders bevorzugt 500 Gew.-ppm bis 7 Gew.% insbesondere 0,1 Gew.-% bis 5 Gew.-%, bezogen auf die Summe aus Aluminiumoxid und solchen Verbindungen, enthalten sein. Weiterhin können neben dem Oxid-Anion weitere Anionen, wie Hydroxid-Anionen, vorliegen.

Falls zusätzlich zu dem Heterogenkatalysator noch ein homogener Katalysator in dem erfindungsgemäßen Verfahren verwendet wird, so handelt es sich um einen, der ausgewählt ist aus der Gruppe der C1- bis C20 -Mono- und Diamine, vorzugsweise der C4- bis C9-Diamine, besonders bevorzugt Hexylamin. Darüber hinaus kann als gegebenenfalls zu verwendender homogener Katalysator eine ionische Flüssigkeit, verwendet werden, die ausgewählt ist aus der Gruppe, bestehend aus Bronstedt-Säure-Adukten von organischen stickstoffhaltigen Substanzen.

In dem erfindungsgemäßen Verfahren können neben dem mindestens einen Heterogenkatalysator auch mehrere homogene Katalysatoren verwendet werden.

In einer besonders bevorzugten Ausführungsform kann das erfindungsgemäße Verfahren zur Isomerisierung in ein Gesamtverfahren integriert sein, in dem man
a) 3-Pentennitril oder eine Mischung, enthaltend 3-Pentennitril, in Gegenwart eines Nickel(0) enthaltenden Katalysators nach an sich bekannten Verfahren zu Adipodinitril hydrocyaniert unter Erhalt von cis-2-Pentennitril als Nebenprodukt,
b) von der Produktmischung cis-2-Pentennitril ganz oder teilweise gegebenenfalls mit anderen Stoffen aus der Hydrocyanierung zusammen abtrennt, beispielsweise durch Destillation,
c) cis-2-Pentennitrif aus Schritt b) nach dem oben beschriebenen erfindungsgemäßen Verfahren isomerisiert unter Erhalt eines Sumpfstroms, enthaltend trans-3-Pentennitril, gegebenenfalls weiteren Verbindungen, die ausgewählt sind aus der Gruppe, bestehend aus trans-2-Pentennitril, 4-Pentennitril und cis-3-Pentennitril, und eines Kopfstroms, enthaltend nicht isomerisiertes cis-2-Pentennitril und gegebenenfalls leichter als trans-3-Pentennitril siedenden Verbinungen, die ausgewählt sind aus der Gruppe, bestehend aus C5-Nitrilen, beispielsweise Z-2-Methyl-2-butennitril, E-2-Methyl-2-butennitril, 2-Methyl-3-butennitril, Valeriansäurenitril sowie andere aus der Hydrocyanierung stammende leichter als trans-3-Pentennitril siedende Komponenten,
d) von dem in Schritt c) erhaltenen Sumpfstrom gegebenenfalls enthaltenes cis-2-Pentennitril abtrennt, beispielsweise durch Destillation, und in Schritt c) zurückführt unter Erhalt eines Reststroms,
e) den in Schritt d) erhaltenen Reststrom in Schritt a) zurückführt.

Der Sumpftstrom aus c) kann einen Restanteil an cis-2-Pentennitril enthalten. Dieser ist vorzugsweise kleiner 10 Gew.-%, besonders bevorzugt kleiner 1 Gew.-%, bezogen auf den Sumpfstrom.

Der Kopfstrom aus c) kann einen Restanteil trans-3-Pentennitril enthalten. Dieser ist vorzugweise kleiner 10 Gew.-%, besonders bevorzugt kleiner 5 Gew.-%, bezogen auf den Kopfstrom.

Vorzugsweise kann man in Schritt a) als Nickel(0) enthaltenden Katalysator einen solchen einsetzen, der neben Nickel(0) weiterhin einen einbindigen oder einen mehrbindigen oder ein Gemisch aus ein- und mehrbindigen Liganden, besonders bevorzugt einen einbindigen und einen Chelatliganden, insbesondere bevorzugt einen Chelatliganden, der mehrere, wie zwei oder drei, zur Bindung an das besagte Nickel(0) fähige dreibindige Phosphoratome, die unabhängig voneinander als Phosphin, Phosphinit, Phosphonit oder Phosphit vorliegen können, aufweist. Besonders vorteilhaft sollte der Katalysator weiterhin eine Lewissäure enthalten. Derartige Katalysatorsysteme sind an sich bekannt.

Eine Möglichkeit zur Erhöhung des Umsatzes ist die Entfernung des Reaktionsproduktes der Isomerisierung, um somit das Gleichgewicht auf die Seite des gewünschten isomerisierten Pentennitrils zu verschieben. Eine Möglichkeit, das isomerisierte Pentennitril aus dem Gleichgewicht zu entfernen, besteht darin, den höheren Siedepunkt des isomerisierten Pentennitrils im Vergleich zu dem zu isomerisierenden Pentennitril auszunutzen.

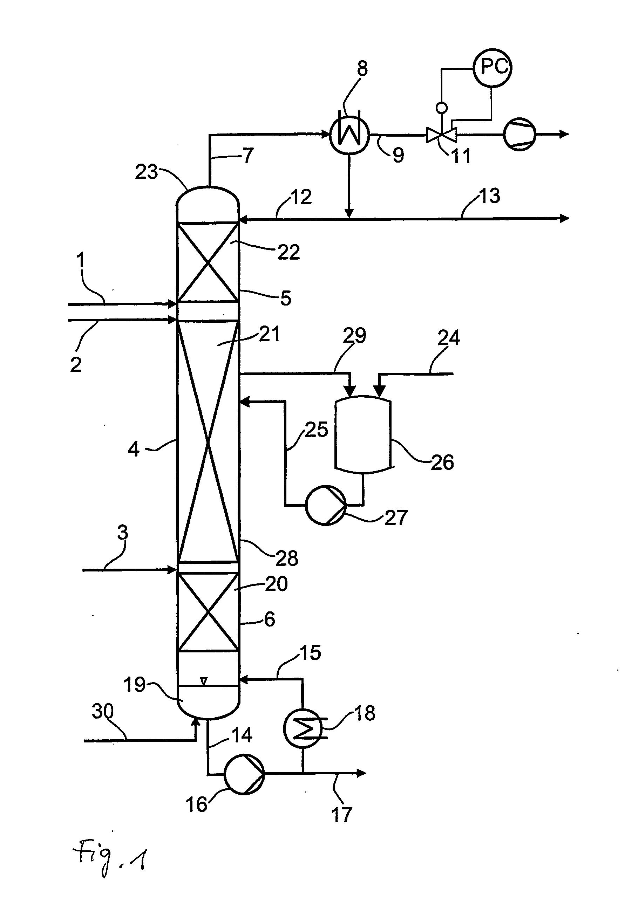

Eine besonders bevorzugte Durchführung des erfindungsgemäßen Verfahrens wird nachfolgend für die Isomerisierung von cis-2-Pentennitril zu trans-3-Pentennitril an einem heterogenen Katalysator anhand der Figur 1 beschrieben:
Das erfindungsgemäße Verfahren wird vorzugsweise so durchgeführt, dass man cis-2-Pentennitril oder ein solches enthaltendes Gemisch über die Zuläufe 1, 2 oder 3 unter-, inner- oder oberhalb des Katalysator enthaltenden Bereichs 21 auf eine als Reaktionskolonne fungierende Destillationskolonne 4 dosiert.

Der Druck und die Temperatur werden so eingestellt, dass sich hohe Reaktionsgeschwindigkeiten bei ausreichend hoher Selektivität ergeben. Dabei wird der Druck gasseitig 9 nach dem Kopfkondensator bevorzugt so eingestellt, dass die Temperatur im Sumpf 19 zwischen 30 °C und 300 °C liegt. Die Einstellung des Drucks kann mit einer Vakuumpumpe 10 und/oder einer Druckregeleinrichtung 11 erfolgen. Figur 1 zeigt die Ausführung mit einer Druckregeleinrichtung 11. An deren Stelle kann z.B. die Vakuumpumpe 10 installiert sein.

Am Katalysator im Bereich der Einbauten 21 der Reaktionskolonne 4 findet die Isomerisierung des cis-2-Pentennitrils statt. Durch die überlagerte Destillation werden die gebildete Reaktionsprodukte dem Reaktionsgleichgewicht und der Reaktionszone kontinuierlich entzogen und gelangen in den Sumpf 19 der Destillationskolonne und werden über den Strom 14 abgezogen. Die überlagerte Destillation sorgt für eine positive Beeinflussung des Reaktionsgleichgewichts in dem Sinn, dass stets cis-2-Pentennitril im Bereich der Reaktionszone 21 angereichert und trans-3-Pentennitril und andere, höher als cis-2-Pentennitril siedende Reaktionsprodukte in der Reaktionszone abgereichert werden und damit für hohe Ausbeuten an trans-3-Pentennitril bezüglich des eingesetzten cis-2-Pentennitrils sorgen.

Das höher siedende Reaktionsprodukt sammelt sich im Sumpf 19 der Destillationskolonne 4 an und wird mittels einer Pumpe 16 gegebenenfalls zusammen mit höher als cis-2-Pentennitril siedenden Nebenprodukten der Isomerisierung sowie bereits im Eduktstrom befindlichen höher als cis-2-Pentennitril siedenden Komponenten über den Sumpfstrom 14 abgezogen. Ein Teil des Sumpfstroms 14 wird mit einem Verdampfer 18 verdampft und über die Brüdenleitung 15 in die Kolonne geführt, der andere Teil 17 wird ausgeschleust. Zur Entfernung von leicht siedenden Komponenten wird zusätzlich ein Inertgas 30 in den Sumpf der Kolonne eingespeist.

Am Kopf 23 der Kolonne 4 reichert sich nicht umgesetztes cis-2-Pentennitril und gegebenenfalls niedriger als cis-2-Pentennitril siedende Komponenten aus dem Eduktstrom, gegebenenfalls zusammen mit leicht siedenden Nebenprodukten der Reaktion, an. Dieses wird über die Leitung 7 in den Kondensator 8 geführt, auskondensiert und über die Leitung 13 ausgeschleust. Ein Teil des Kondensats wird als Rücklauf 12 wieder auf die Kolonne gegeben.

Hierbei handelt es sich bevorzugt um mehr als 30%, besonders bevorzugt um mehr als 90 % des Kondensats. Auf diese Weise kann durch den internen Rücklauf in der Kolonne ein vorteilhaftes Konzentrationsprofil eingestellt werden.

Bevorzugt wird das Konzentrationsprofil in der Kolonne durch den Energieeintrag und das Rücklaufverhältnis so eingestellt, dass sich eine Anreicherung des cis-2-Pentennitrils am oberen Ende des Reaktionsbereiches 21 gegenüber dem unteren Ende des Reaktionsbereichs ausbildet. So kann eine hohe Konzentration des leicht siedenden Reaktanden im Reaktionsbereich eingestellt werden, die zu hohen Selektivitäten des bei niedrigen lokalen Umsätzen kinetisch bevorzugten Reaktionsproduktes trans-3-Pentennitril führt. Es wird ein Rücklaufverhältnis zwischen 2 und 500 eingestellt.

Auf den Kolonneneinbauten der Reaktionszone 21 oberhalb der Zulaufstelle 3 der Kolonne 4 erfolgt im Wesentlichen die Umsetzung des Eduktstroms mit simultaner destillativer Entfernung der entstehenden Produkte.

In der unteren Trennzone 6 mit den Einbauten 20 wird das schwer siedende Reaktionsprodukt weitgehend von leicht siedenden Komponenten abgetrennt. In der oberen Trennzone 5 mit den Einbauten 22 werden leicht siedende Nebenkomponenten weitgehend von schwer siedenden Komponenten abgetrennt. Bei optimaler Kolonnenkonfiguration kann so das gesamte cis-2-Pentennitril ohne zusätzlichen Reaktor umgesetzt werden und das gesamte trans-3-Pentennitril im Sumpf ohne zusätzliche Trennvorrichtung erhalten werden.

Die Katalysatorpackung der Destillationskolonne in der Reaktionszone 21 besteht aus losen Katalysatorteilchen, die unter der Einwirkung der Schwerkraft eingebracht und verteilt werden und bei Bedarf wieder ausgetragen werden können. Dabei weist die Katalysatorpackung zwei unterschiedliche Teilbereiche auf, wobei im ersten Teilbereich der Packung die Katalysatorteilchen lose unter der Einwirkung der Schwerkraft eingebracht und verteilt werden und bei Bedarf wieder ausgetragen werden können und im zweiten Teilbereich jedoch aufgrund der geometrischen Verhältnisse im Vergleich zum Katalysatorteilchen keine Katalysatorteilchen eingebracht werden können.

Zur Erhöhung der Verweilzeit der Reaktionszone ist es möglich, einen Teilstrom durch einen oder mehrere Seitenabzüge 29 aus der Destillationskolonne 4 durch den oder die Behälter 26 zu leiten und mit Hilfe je einer Pumpe 27, die diese Behälter verlassenden Teilströme 25 wieder in die Destillationskolonne 4 zurückzuführen. Die Behälter sind dabei mit heterogenen Katalysatoren gefüllt. Ferner ist eine Beheizung der Behälter 26 gegeben. Zusätzlich kann weiterer cis-2-Pentennitril haltiger Zulauf 24 in einen oder mehrere Behälter 26 eingespeist werden.

In den Trennzonen 5 ,6 sind Kolonneneinbauten 20, 22 mit hoher Trennstufenzahl angeordnet.

Die Wärmezufuhr in das Reaktionssystem und gegebenenfalls in dem Behälter oder in den Behältern 26 erfolgt nicht nur über den Verdampfer 18, sondern zusätzlich über außenliegende Wärmetauscher 28 oder über sich direkt auf dem Kolonneneinbauten 20, 21 und/oder 22 befindliche Wärmetauscher. Ferner ist es möglich, aus der Kolonne an beliebigen Stellen über Seitenabzüge Teilströme abzuziehen. So ist es z.B. möglich, die Kolonne unter totalem Rücklauf zu betreiben und über einen Seitenabzug unterhalb der Katalysatorpackung, aber oberhalb des Zulaufs 3 Zwischensieder im Siedebereich zwischen cis-2-Pentennitril und trans-3-Pentennitril auszuschleusen. Der Zulauf 3 liegt dabei bevorzugt innerhalb oder unterhalb der Trennzone 6.

Die vorliegende Erfindung wird anhand der folgenden Beispiele näher erläutert:

### Ausführungsbeispiele

### Beispiel 1:

### Heterogen katalysierte Isomerisierung von cis-2-Pentennitril

### A. Beschreibung der Apparatur

Die Versuchsapparatur besteht aus einem beheizbaren, mit Rührer ausgestatteten 2-Liter-Reaktionskolben aus Edelstahl, auf den eine Destillationskolonne (Länge: 1,2 m Durchmesser: 55 mm) aufgesetzt ist. Die Kolonne ist im unteren Bereich (Trennzone 20) mit 7 Segmenten einer strukturierten Gewebepackung vom Typ Montz A3-500 (Gesamthöhe: 35 cm) und im oberen Bereich (Trennzone 22) mit 1 Segment einer strukturierten Gewebepackung vom Typ Montz A3-500 (Gesamthöhe: 5 cm) gefüllt. Die Reaktionszone 21 im mittleren Bereich der Kolonne wurde mit Katalysatorpackungen bestückt, in die die Katalysatorteilchen lose unter der Einwirkung der Schwerkraft eingebracht, verteilt und wieder ausgetragen sind, wobei die Katalysatorpackung erste und zweite Teilbereiche aufweist, wobei im ersten Teilbereich der Packung die Katalysatorteilchen lose unter der Einwirkung der Schwerkraft eingebracht, verteilt und wieder ausgetragen sind, und im zweiten Teilbereich jedoch aufgrund der geometrischen Verhältnisse im Vergleich zum Katalysatorteilchen keine Katalysatorteilchen eingebracht werden können. Ingesamt werden in die Packung ca. 800 g des Katalysators gefüllt. Als Katalysator werden Stränge aus Al₂O₃ verwendet. Die Kolonne ist in regelmäßigen Abständen mit Thermoelementen bestückt, so dass außer im Sumpf und am Kopf der Kolonne an jeder 3. bis 4. theoretischen Stufe die Temperatur gemessen werden kann. Zusätzlich zum Temperaturprofil kann mit Hilfe entsprechender Probennahmestellen das Konzentrationsprofil in der Kolonne ermittelt werden.

Die Reaktanden werden aus auf Waagen stehenden Vorlagebehältern mit einer Pumpe massen-stromgeregelt in die Kolonne dosiert. Der Verdampfer 18, der mit Hilfe eines Thermostaten auf 177 °C beheizt wird, hat während des Betriebs je nach Verweilzeit ein Hold-up zwischen 50 und 150 ml. Der Sumpfstrom 17 wird aus dem Verdampfer mit einer Pumpe standgeregelt in einen auf einer Waage stehenden Behälter gefördert. Der Kopfstrom 7 der Kolonne wird in einem Kondensator (8), der mit einem Kryostaten betrieben wird, auskondensiert und vollständige 12 als Rücklauf auf die Kolonne gegeben. Die Apparatur ist mit einer Druckregelung 11 ausgestattet und auf einen Systemdruck von 20 bar ausgelegt. Alle ein- und austretenden Stoffströme werden während des gesamten Versuchs mit einem PLS kontinuierlich erfasst und registriert. Die Apparatur wird im 24-Stunden-Betrieb gefahren (Stationarität).

### B. Versuchsdurchführung

Direkt oberhalb der Katalysatorpackung werden kontinuierlich 100 g/h cis-2-Pentennitril (97,6 Gew.-%, Rest andere C5-Nitrile) in die Kolonne dosiert. Als Katalysator in der Reaktionszone 21 wurden Stränge aus Al₂O₃ verwendet. Es wird ein Systemdruck von 1 bar und ein Rücklaufverhältnis, bezogen auf den Zulauf, von 10 kg/kg eingestellt. Die Sumpftemperatur beträgt 149 °C. Als Sumpfstrom der Kolonne wird 100 g/h Produkt mit 4,3 Gew.% cis-3-Pentennitril, 50,8 Gew.% trans-2-Pentennitril, 32,3 Gew.-% trans-3-Pentennitril und 10,9 Gew.% cis-3-Pentennitril sowie sonstige Nebenkomponenten (Hochsieder) abgezogen. Insgesamt werden 95,6 % des eingesetzten cis-2-Pentennitrils umgesetzt.

### Beispiel 2:

Heterogen katalysierte Isomerisierung von cis-2-Pentennitril in einem Gemisch mit trans-3-Pentennitril.

### A. Beschreibung der Apparatur

### Siehe Beispiel 1.

### B. Versuchsdurchführung

Unterhalb der Katalysatorpackung werden kontinuierlich 100 g/h eines Gemischs aus trans-3-Pentennitril (47,5 Gew.-%) und cis-3-Pentennitril (47, 2 Gew.-%) sowie sonstigen Stoffen aus der Herstellung von Adipodinitril mittels Hydrocyanierung von 1,3-Butadien in die Kolonne dosiert. Als Katalysator in der Reaktionszone 21 werden Stränge aus Al₂O₃ verwendet. Es wird ein Systemdruck von 1 bar und ein Rücklaufverhältnis bezogen auf den Zulauf, von 10 kg/kg eingestellt. Die Sumpftemperatur beträgt 149 °C. Als Sumpfstrom der Kolonne werden 100 g/h Produkt mit 4,9 Gew.-% cis-2-Pentennitril, 20,1 Gew.-% trans-2-Pentennitril, 62,3 Gew.-% trans-3-Pentennitril und 6,6 Gew.-% cis-3-Pentennitril sowie sonstige Nebenkomponenten und Hochsieder abgezogen. Insgesamt wird 89,7 % des eingesetzten cis-2-Pentennitrils umgesetzt.

## Patentansprüche

1. Verfahren zur Isomerisierung von Pentennitril in einem Eduktstrom, **dadurch gekennzeichnet, dass** die Isomerisierung an mindestens einem heterogenen Katalysator, ausgewählt aus der Gruppe der Oxide der 3. oder 4. Hauptgruppe oder der Oxide der 3. oder 4. Nebengruppe des Periodensystems der Elemente in einer Destillationskolonne, mindestens umfassend eine Sumpfzone, eine Reaktionszone und eine Kopfzone, stattfindet und dass während der Isomerisierung das Isomerisierungsedukt im Verhältnis zu dem Isomerisierungsprodukt in der Reaktionszone der Destillationskolonne destillativ angereichert wird.

2. Verfahren nach Anspruch 1, **dadurch gekennzeichnet, dass** cis-2-Pentennitril zu trans-3-Pentennitril isomerisiert wird.

3. Verfahren nach Anspruch 2, **dadurch gekennzeichnet, dass** das trans-3-Pentennitril im Sumpf der Destillationskolonne und das cis-2-Pentennitril im Kopf der Destillationskolonne erhalten werden.

4. Verfahren nach einem der Ansprüche 1 bis 3, **dadurch gekennzeichnet, dass** als heterogener Katalysator Aluminiumoxid verwendet wird.

5. Verfahren nach einem der Ansprüche 1 bis 4, **dadurch gekennzeichnet, dass** als heterogener Katalysator Aluminiumoxid verwendet wird, das Siliciumdioxid, Titandioxid, Eisenoxid, Alkalioxide, Erdalkalioxide, seltene Erdenoxide oder deren Gemische enthält.

6. Verfahren nach einem der Ansprüche 1 bis 5, **dadurch gekennzeichnet, dass** der heterogene Katalysator in eine Packung eingebracht wird, die im Kolonneninnenraum Zwischenräume ausbildet, wobei der Quotient aus dem hydraulischen Durchmesser für den Gasstrom durch die Packung oder Füllkörper und dem äquivalenten Durchmesser der Katalysatorteilchen 2 bis 20 beträgt, so dass die Katalysatorteilchen lose unter der Einwirkung der Schwerkraft in die Zwischenräume eingebracht, verteilt und bei Bedarf wieder ausgetragen werden können, und andere Zwischenräume ausbildet, in denen der Quotient aus dem hydraulischen Durchmesser für den Gasstrom durch die Packung oder die Füllkörper und dem äquivalenten Durchmesser der Katalysatorteilchen kleiner als 1 ist, so dass in die zweiten Teilbereiche keine Katalysatorteilchen eingebracht werden.

7. Verfahren nach einem der Ansprüche 1 bis 6, **dadurch gekennzeichnet, dass** die Isomerisierung zusätzlich in Gegenwart eines homogenen Katalysators durchgeführt wird.

8. Verfahren nach Anspruch 7, **dadurch gekennzeichnet, dass** die Umsetzung in Gegenwart einer ionischen Flüssigkeit als Homogenkatalysator durchgeführt wird, wobei die ionische Flüssigkeit ausgewählt aus der Gruppe, bestehend aus Brönstedt-Säure-Adukten von organischen stickstoffhaltigen Substanzen.

9. Verfahren nach Anspruch 7, wobei als Katalysator ein C1- bis C20-Mono- oder - Diamin eingesetzt wird.

10. Verfahren nach einem der Ansprüche 1 bis 9, **dadurch gekennzeichnet, dass** der Eduktstrom weitere Komponenten, ausgewählt aus der Gruppe, bestehend aus C5-Mononitrilen, C6-Dinitrilen, aliphatischen C1- bis C16-Alkanen, cyclischen C1- bis C16-Alkanen, aliphatischen C1- bis C16-Alkenen, cyclischen C1- bis C16-Alkenen, enthält.

11. Verfahren nach einem der Ansprüche 1 bis 9, **dadurch gekennzeichnet, dass** der Eduktstrom aus einem Verfahren zur Hydrocyanierung von 3-Pentennitril stammt.

12. Verfahren nach einem der Ansprüche 1 bis 11, **dadurch gekennzeichnet, dass** die Temperatur in der Sumpfzone der Destillationskolonne 30°C bis 300°C beträgt.

## Claims

1. A process for isomerizing pentenenitrile in a reaction stream, wherein the isomerization takes place over at least one heterogeneous catalyst, selected from the group of oxides of main group 3 or 4 or of oxides of transition group 3 or 4 of the Periodic Table of Elements, in a distillation column at least comprising a bottom zone, a reaction zone and a top zone, and, during the isomerization, the isomerization reactant is distillatively enriched in the reaction zone of the distillation column in relation to the isomerization product.

2. The process according to claim 1, wherein cis-2-pentenenitrile is isomerized to trans-3-pentenenitrile.

3. The process according to claim 2, wherein the trans-3-pentenenitrile is obtained in the bottom of the distillation column and the cis-2-pentenenitrile in the top of the distillation column.

4. The process according to any of claims 1 to 3, wherein the heterogeneous catalyst used alumina.

5. The process according to any of claims 1 to 4, wherein the heterogeneous catalyst used is alumina which comprises silicon dioxide, titanium dioxide, iron oxide, alkali metal oxides, alkaline earth metal oxides, rare earth oxides or mixtures thereof.

6. The process according to any of claims 1 to 5, wherein the heterogeneous catalyst is introduced into a structured packing which forms interstices in the column interior, the quotient of the hydraulic diameter for the gas stream through the structured packing or random packing and the equivalent diameter of the catalyst particles being from 2 to 20, so that the catalyst particles can be loosely inserted into the interstices under the action of gravity, distributed and discharged again if required, and the structured packing forms other interstices in which the quotient of the hydraulic diameter for the gas stream through the structured packing or the random packing and the equivalent diameter of the catalyst particles is less than 1, so that no catalyst particles can be inserted into the second subregions.

7. The process according to any of claims 1 to 6, wherein the isomerization is additionally carried out in the presence of a homogeneous catalyst.

8. The process according to claim 7, wherein the reaction is carried out in the presence of an ionic liquid as a homogeneous catalyst, the ionic liquid being selected from the group consisting of Bronsted acid adducts of organic nitrogen-containing substances.

9. The process according to claim 7, wherein the catalyst used is a C1- to C20-mono- or -diamine.

10. The process according to any of claims 1 to 9, wherein the reactant stream comprises further components selected from the group consisting of C5-mononitriles, C6-dinitriles, aliphatic C1- to C16-alkanes, cyclic C1- to C16-alkanes, aliphatic C1- to C16-alkenes, cyclic C1- to C16-alkenes.

11. The process according to any of claims 1 to 9, wherein the reactant stream stems from a process for hydrocyanating 3-pentenenitrile.

12. The process according to any of claims 1 to 11, wherein the temperature in the bottom zone of the distillation column is from 30°C to 300°C.

## Revendications

1. Procédé d'isomérisation de pentène-nitrile dans un courant de réactifs, **caractérisé en ce que** l'isomérisation a lieu sur au moins un catalyseur hétérogène, choisi dans le groupe des oxydes du groupe principal 3 ou 4 ou des oxydes du groupe de transition 3 ou 4 du tableau périodique des éléments, dans une colonne de distillation, comprenant au moins une zone de fond, une zone de réaction et une zone de tête, et **en ce que**, pendant l'isomérisation, le réactif d'isomérisation s'accumule par distillation par rapport au produit d'isomérisation dans la zone de réaction de la colonne de distillation.

2. Procédé selon la revendication 1, **caractérisé en ce que** du cis-2-pentène-nitrile est isomérisé en trans-3-pentène-nitrile.

3. Procédé selon la revendication 2, **caractérisé en ce que** le trans-3-pentène-nitrile est obtenu dans le fond de la colonne de distillation et le cis-2-pentène-nitrile dans la tête de la colonne de distillation.

4. Procédé selon l'une quelconque des revendications 1 à 3, **caractérisé en ce que** de l'oxyde d'aluminium est utilisé en tant que catalyseur hétérogène.

5. Procédé selon l'une quelconque des revendications 1 à 4, **caractérisé en ce que** de l'oxyde d'aluminium qui contient du dioxyde de silicium, du dioxyde de titane, de l'oxyde de fer, des oxydes alcalins, des oxydes alcalino-terreux, des oxydes de terres rares ou leurs mélanges est utilisé en tant que catalyseur hétérogène.

6. Procédé selon l'une quelconque des revendications 1 à 5, **caractérisé en ce que** le catalyseur hétérogène est introduit dans un garnissage qui forme des espaces intermédiaires dans l'espace intérieur de la colonne, le quotient entre le diamètre hydraulique pour le courant gazeux au travers du garnissage ou des corps de remplissage et le diamètre équivalent des particules de catalyseur étant de 2 à 20, de sorte que les particules de catalyseur puissent être introduites en vrac sous l'effet de la pesanteur dans les espaces intermédiaires, réparties et au besoin de nouveau déchargées, et forme d'autres espaces intermédiaires, dans lesquels le quotient entre le diamètre hydraulique pour le courant gazeux au travers du garnissage ou des corps de remplissage et le diamètre équivalent des particules de catalyseur est inférieur à 1, de sorte qu'aucune particule de catalyseur ne soit introduite dans les secondes sections.

7. Procédé selon l'une quelconque des revendications 1 à 6, **caractérisé en ce que** l'isomérisation est également réalisée en présence d'un catalyseur homogène.

8. Procédé selon la revendication 7, **caractérisé en ce que** la réaction est réalisée en présence d'un liquide ionique en tant que catalyseur homogène, le liquide ionique étant choisi dans le groupe constitué par les adduits d'acides de Bronsted de substances organiques contenant de l'azote.

9. Procédé selon la revendication 7, dans lequel une mono- ou diamine en C1 à C20 est utilisée en tant que catalyseur.

10. Procédé selon l'une quelconque des revendications 1 à 9, **caractérisé en ce que** le courant de réactifs contient des composants supplémentaires choisis dans le groupe constitué par les mononitriles en C5, les dinitriles en C6, les alcanes aliphatiques en C1 à C16, les alcanes cycliques en C1 à C16, les alcènes aliphatiques en C1 à C16, les alcènes cycliques en C1 à C16.

11. Procédé selon l'une quelconque des revendications 1 à 9, **caractérisé en ce que** le courant de réactifs est issu d'un procédé d'hydrocyanation de 3-pentène-nitrile.

12. Procédé selon l'une quelconque des revendications 1 à 11, **caractérisé en ce que** la température dans la zone de fond de la colonne de distillation est de 30°C à 300°C.
